# EUROPÄISCHE PATENTSCHRIFT

(11) **EP 0 915 680 B1**
(45) Veröffentlichungstag und Bekanntmachung des Hinweises auf die Patenterteilung: **23.10.2002**
(21) Anmeldenummer: 98913498.6
(22) Anmeldetag: 21.04.1998
(51) Int. Cl.: A61B 17/16

(54) **VORRICHTUNG ZUR HERSTELLUNG VON ENDOCHONDRALEN ODER OSTEOCHONDRALEN BOHRUNGEN**
DEVICE FOR PRODUCING ENDOCHONDRAL AND OSTEOCHONDRAL DRILLED HOLES
DISPOSITIF POUR REALISER DES TROUS ENDOCHONDRAUX ET OSTEOCHONDRAUX

(30) Priorität: 25.04.1997 CH 97397
(43) Veröffentlichungstag der Anmeldung: 19.05.1999
(73) Patentinhaber: Sulzer Orthopädie AG, 6340 Baar (CH)
(72) Erfinder: ZECH, Manfred, CH-8450 Andelfingen (CH); SAAGER, Christoph, CH-3055 Frieswil (CH); MAINIL-VARLET, Pierre, CH-3005 Bern (CH); MÜLLER-GLAUSER, Werner, CH-8542 Wiesendangen (CH)
(74) Vertreter: Manitz, Finsterwald & Partner Gbr
(86) Internationale Anmeldenummer: CH9800154
(87) Internationale Veröffentlichungsnummer: WO98048707

(56) Entgegenhaltungen:
- SOVIET PATENTS ABSTRACTS Section PQ, Week 9202 26.Februar 1992 Derwent Publications Ltd., London, GB; Class P31, AN 92-015519 XP002069179 -& SU 1 644 923 A (KRYMSK MED INST ) , 30.April 1991

## Beschreibung

Die Erfindung liegt auf dem Gebiet der Medizinaltechnik und betrifft eine Vorrichtung gemäss dem Oberbegriff des ersten Patentanspruchs zur Herstellung von endochondralen und von osteochondralen Bohrungen.

Es sind verschiedenste künstliche und in vitro gezüchtete Implantate bekannt, mit denen Defekte in Gelenkknorpeln oder in Gelenkknorpeln und darunter liegendem Knochengewebe reparierbar sind. Derartige Implantate sind beispielsweise verformbare Massen oder Pasten, die in die Defekte gestrichen werden und sich im wesentlichen jeder Defektform anpassen, die aber in den meisten Fällen keine genügende mechanische Stabilität aufweisen, um nach dem Eingriff sofort belastet werden zu können.

Es sind auch Implantate bekannt, die mechanische Eigenschaften besitzen, die denen von natürlichem Knorpel bzw. Knochen mindestens ähnlich sind. Derartige Implantate haben den Vorteil, dass sie die mechanische Funktion des zu ersetzenden Gewebes (Knorpel oder Knorpel und Knochen) sofort übernehmen können, das heisst sofort nach dem Eingriff mindestens beschränkt belastbar sind. Derartige Implantate oder auch Transplantate haben aber den Nachteil, dass sie in ihrer Form an die Form des Defektes angepasst werden müssen oder dass der Defekt vor der Implantation zu einer vorbestimmten Form ausgeschnitten oder ausgebohrt werden muss.

Es zeigt sich, dass eine möglichst genaue Anpassung der Implantatform an die Defektform für den Heilungsprozess vorteilhaft ist. Aus diesem Grunde ist es üblich, bei einem entsprechenden chirurgischen Eingriff die defekte Stelle auszuschneiden zu einer Vertiefung mit einer möglichst definierten Form und ein Implantat zu verwenden, das dieselbe Form aufweist.

Bei Transplantationen von Knorpelgewebe oder Knorpel- und Knochengewebe von gesunden Stellen an defekte Stellen, ist es beispielsweise üblich, eine Stanzklinge mit einer kreisrunden Schneidekante in das Knorpelgewebe und das darunterliegende Knochengewebe zu drehen, durch Rütteln an der Klinge die ausgestanzte Säule an ihrem Grund abzubrechen und mit der Klinge zu entfernen. Diese Methode wird sowohl für das Ausschneiden von Defekten als auch für die Herstellung entsprechender Gewebesäulen für die Transplantation angewendet.

Eine Trennung von Gewebe durch das oben genannte Rütteln einer Stanzklinge ist nur möglich im Knochengewebe. Das heisst mit anderen Worten, dass diese Methode auf osteochondrale "Bohrungen" beschränkt bleibt oder dass Defekte, die nur die Knorpelschicht betreffen, mangels einer Methode zur Herstellung von endochondralen Bohrungen zu osteochondralen Öffnungen vertieft werden müssen. Dieser Nachteil fällt bei der Verwendung von mit der gleichen Methode hergestellten Gewebesäulen als Transplantaten nicht ins Gewicht, ist aber beispielsweise bei der Implantation von in vitro gezüchtetem Knorpel ein Nachteil.

Ferner zeigt es sich, dass die nach der genannten Methode durch Ausstanzen und Brechen hergestellten Säulen und Öffnungen undefinierte Grundflächen und Höhen bzw. Tiefen mit relativ grossen Schwankungen aufweisen, wodurch die Passgenauigkeit im Bereich dieser Grundflächen sowohl bei Transplantaten als auch bei Implantaten weitgehend dem Zufall überlassen bleibt oder durch eine plastische Masse verbessert werden muss. Aus demselben Grund ist es mit der bekannten Methode auch schwierig, die Oberflächen von Transplantat bzw. Implantat und Umgebung genau aufeinander auszurichten, was für den Erfolg des Eingriffs von Wichtigkeit wäre.

Aus der SU-A-1644923 ist Vorrichtung zum Schneiden von Knochengewebe bekannt, die einen Schraubenbohrer aufweist, der sich in einem hohlen Schneidwerkzeug befindet. Der Schaft des Bohrers ist mit dem Schneidwerkzeug über ein Reduziergetriebe verbunden. Das sich drehende Schneidwerkzeug entfernt die Produkte des Schneidprozesses sowie das Knochenmark.

Die Erfindung stellt sich nun die Aufgabe, eine Vorrichtung zu schaffen, mit der endochondrale und osteochondrale Defekte bei einer minimalen Vergrösserung aufbohrbar sind zu endochondralen bzw. osteochondralen Bohrungen mit einer möglichst genau definierten Tiefe und einer möglichst genau flachen Grundfläche, derart, dass sie mit entsprechenden in vitro gezüchteten oder künstlichen Implantaten oder auch von Transplantaten einer definierten Form genau gefüllt werden können.

Diese Aufgabe wird gelöst durch die Vorrichtung, wie sie in den Patentansprüchen definiert ist.

Die erfindungsgemässe Vorrichtung umfasst eine Kombination einer im wesentlichen hohlzylindrischen Hülse, deren eines Ende als z.B. kreisrunde Schneidekante ausgebildet ist, mit einem Flachbohrer, der in der Hülse drehbar und axial verschiebbar angordnet ist. Durch Eindrücken der Schneidekante in das Gewebe, vorteilhafterweise nur in das Knorpelgewebe, wird eine Gewebesäule ausgestanzt, die dann durch Einschrauben des Flachbohrers zerspant und entfernt wird. Dabei wird bis auf die Tiefe der Schneidekante gebohrt oder tiefer, wobei in jeder Tiefe eine geschnittene und genau flache Bohrungsgrundfläche entsteht. Diese Bohrungsgrundfläche weist immer dieselbe Qualität auf, unabhängig davon, ob sie innerhalb des Gelenkknorpels oder im Knochengewebe situiert ist. Der Bohrer wird von Hand oder durch eine geeignete Bohrmaschine angetrieben.

Anhand der folgenden Figuren werden beispielhafte Ausführungsformen der erfindungsgemässen Vorrichtung mehr im Detail beschrieben. Dabei zeigen:
- Figur 1: eine erste beispielhafte Ausführungsform der erfindungsgemässen Vorrichtung, entlang der Achse geschnitten,
- Figur 2 und Figur 3: weitere beispielhafte Ausführungsformen des distalen Bohrerendes,
- Figur 4: eine weitere beispielhafte Ausführungsform des Bohrerschaftes, entlang der Achse geschnitten,
- Figur 5: eine weitere beispielhafte Ausführungsformen des Hülsenendes mit Schneidekante entlang der Achse geschnitten,
- Figur 6: das Anschlagstück der Figur 5 als Draufsicht,
- Figur 7: eine weitere beispielhafte Ausführungsform des Hülsenendes mit Schneidekante.

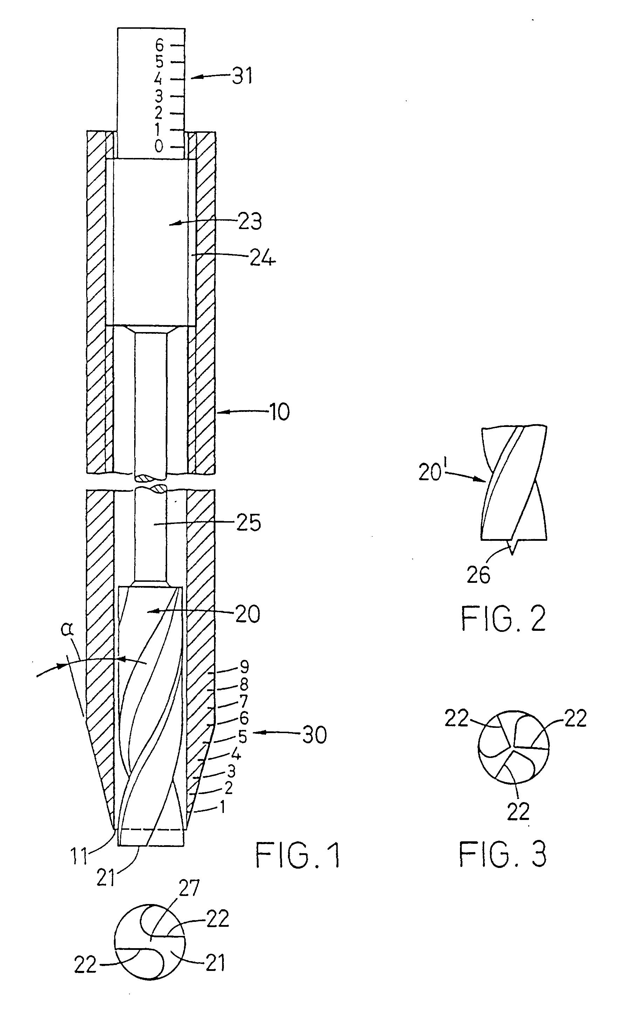

Figur 1 zeigt eine erste, beispielhafte Ausführungsform der erfindungsgemässen Vorrichtung. Diese besteht im wesentlichen aus einer Hülse 10 und aus einem in der Hülse 10 angeordneten Flachbohrer 20. Die Hülse 10 ist im wesentlichen hohlzylindrisch, hat mindestens im Bereich ihres einen Endes einen Innenhohlraum mit einem konstanten Durchmesser und läuft an diesem Ende zu einer kreisrunden Schneidekante 11 aus. Der Flachbohrer 20 hat einen Durchmesser, der derart auf das genannte Ende der Hülse 10 abgestimmt ist, dass er im wesentlichen reibungslos mindestens in diesem Bereich der Hülse 10 drehbar und in axialer Richtung verschiebbar ist.

Der Flachbohrer 20 ist derart in der Hülse 10 angeordnet, dass sein distales, flaches Ende 21, auf dem mindestens zwei Hauptschneiden 22 liegen (in der Draufsicht des flachen Endes 21 sichtbar), gegen das Hülsenende mit der Schneidekante 11 gerichtet ist und im Bereich dieser Schneidekante durch mit Drehung verbundene axiale Verschiebung in Positionen innerhalb der Hülse 10 und ausserhalb der Hülse bringbar ist. Für diese Drehverschiebung ist beispielsweise ein den Bohrer axial verlängernder, durch die Hülse reichender Bohrerschaft 23 vorgesehen, der mit einem Gewinde 24 versehen ist, das in ein entsprechendes Innengewinde in der Hülse 10 eingeschraubt ist. Zwischen dem Gewinde 24 und dem Flachbohrer 20 weist der Bohrerschaft 23 einen Bereich 25 mit einem geringeren Durchmesser auf, wobei die axiale Ausdehnung dieses Bereiches 25 mindestens so gross ist, dass das zerspante Material einer ausgebohrten Gewebesäule zwischen der Hülse 10 und dem dünneren Bereich 25 des Bohrerschafts 23 reichlich Platz findet.

An der Aussenseite der Hülse 10 im Bereich der Schneidekante 11 und im Bereich des entgegengesetzten Endes des Bohrerschafts 23 sind Markierungen 30 und 31 (beispielsweise mit Abständen von 0.1 mm) vorgesehen, anhand derer einerseits die Eindringtiefe der Schneidekante 11 im Gewebe und andererseits die Position des flachen, distalen Bohrerendes 21 relativ zur Schneidekante 11 ablesbar sind. Eine Addition der beiden Positionen ergibt eine Angabe einer erreichten Bohrungstiefe.

Zum Herstellen einer endochondralen oder osteochondralen Bohrung wird die Schneidekante 11 im Bereich des Defektes auf der Gewebeoberfläche positioniert, während das distale Bohrerende 21 so weit in die Hülse 10 geschraubt ist, dass sein Abstand von der Schneidekante 11 etwa der gewünschten Bohrungstiefe entspricht oder grösser ist als diese. Dann wird die Schneidekante 11 in das Gewebe gedrückt, eventuell unter leichter Rotation, derart, dass sie sich bis zur gewünschten Bohrungstiefe in das Gewebe absenkt oder zu einer Tiefe, die kleiner ist als die gewünschte Bohrungstiefe (für osteochondrale Bohrungen beispielsweise bis auf die Grenze zwischen Knorpelgewebe und Knochengewebe).

Durch das Einpressen der Schneidekante 11 in das Gewebe wird eine Gewebesäule ausgestanzt. Die Schnittiefe ist an der Hülsenmarkierung 30 ablesbar.

Die ausgestanzte Gewebesäule wird durch nachfolgendes Einschrauben des Flachbohrers 20 zerspant und das ausgebohrte Material wird durch den Bohrer in die Hülse 10 gefördert. Der Bohrer 20 wird mindestens bis zu einer Position vorgeschraubt, in der sein distales Ende 21 mit der Schneidekante 11 fluchtet (Nullpunkt der Markierung 31 auf dem Bohrerschaft 23), damit mindestens die ganze ausgestanzte Gewebesäule zerspant und entfernt wird. Soll tiefer gebohrt werden, wird der Bohrer 20 weiter vorgeschraubt, wobei er in der Hülse 10 durch das Gewinde 24 genau geführt ist.

Für das Einschrauben des Bohrers von Hand ist am aus der Hülse 10 ragenden Teil des Bohrerschaftes vorteilhafterweise ein nicht dargestellter Handgriff oder mindestens eine verdickte und gegebenenfalls gerändelte Stelle vorgesehen.

Durch die flach angeordneten Hauptschneiden 22 des distalen Bohrerendes 21 entsteht in jeder Tiefe eine flache und sauber geschnittene Bohrungsgrundfläche.

Die Hülse 10 besteht aus einem korrosionsbeständigen, sterilisierbaren Material (z.B. Chromnickelstahl) und hat beispielsweise eine Wandstärke von ca. 1 mm. Die Schneidekante weist vorteilhafterweise einen Schneidewinkel α von ca. 8° auf.

Der Flachbohrer 20 ist beispielsweise ein im Handel erhältlicher Medinzinalbohrer, der durch Umschleifen seines distalen Endes zu einem Flachbohrer gemacht wird. Medizinalbohrer, wie sie für Massnahmen an Knochen üblich sind, bestehen aus einem sterilisierbaren, korrosionsresistenten Material und weisen einen längeren Drall (kleinerer Drallwinkel) auf als Stahlbohrer. Zwischen dem Bohrer 20 und der Innenoberfläche der Hülse 10 ist ein Spiel von ca. 2 x 0,05 mm vorzusehen.

Für die eingangs erwähnten Anwendungen sind Bohrer mit Durchmessern von beispielsweise 3 mm oder 5 mm geeignet. Es können aber ohne weiteres auch Vorrichtungen mit grösseren Bohrerdurchmessern realisiert werden.
Für die eingangs erwähnten Anwendungen ist eine axiale Bohrerverschiebung in der Hülse von ca 15mm genügend. Die axiale Länge der ganzen Vorrichtung beträgt beispielsweise ca. 150 bis 170mm.

Figuren 2 und 3 zeigen zwei weitere distale Bohrerenden, mit denen die Qualität der Grundfläche der Bohrung noch verbessert werden kann. In Figur 2 ist als Seitenansicht das distale Ende eines Flachbohrers 20' dargestellt, der auf seiner sonst flachen Stirnseite im Bereich der Bohrerseele eine Dorn 26 aufweist. Dieser Dorn, der vorteilhafterweise einen Spitzenwinkel von ca. 50° aufweist, wird durch die Bohrbewegung in die Grundfläche einer Bohrung getrieben.

Dadurch kann sichergestellt werden, dass diese Grundfläche keine mittige Erhöhung aufweist, wie sie bei Verwendung eines Bohrers gemäss Figur 1 durch die nichtschneidende Querschneide 27 entstehen kann. Für den gleichen Zweck kann auch ein Bohrer mit drei Hauptschneiden 22, wie er in der Figur 3 dargestellt ist, verwendet werden. Ein derartiger Bohrer weist keine Querschneide auf.

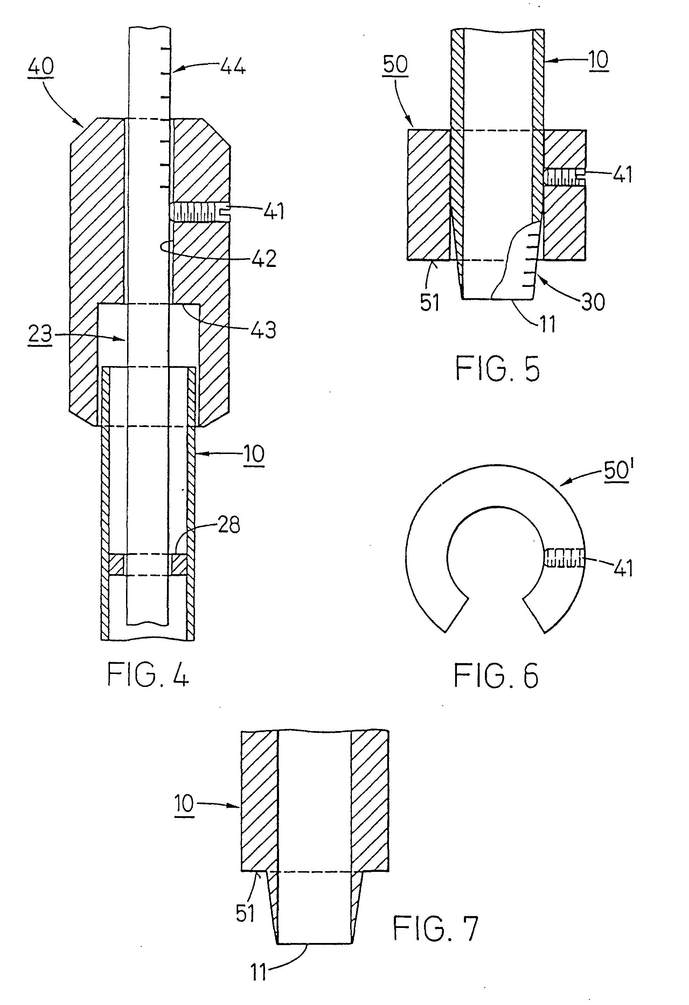

Figur 4 zeigt eine weitere Ausführungsform desjenigen Endes des Bohrerschaftes 23, das auf der der Schneidekante entgegengesetzten Seite aus der Hülse 10 ragt. Im Bereich dieses Endes ist auf dem Bohrerschaft 23 beispielsweise mit Hilfe einer Fixierschraube 41 ein Haltestück 40 mit einer durchgehenden Bohrung 42 montiert. Die Bohrung 42 des Haltestückes 40 hat auf der der Hülse 10 zugewandten Seite einen grösseren Durchmesser als auf der anderen Seite, wodurch ein Anschlag 43 gebildet wird. Der Anschlag 43 ist derart dimensioniert, dass der Bohrer bzw. der Bohrerschaft 23 mit darauf montiertem Haltestück 40 nur so weit in die Hülse 10 vorgeschoben werden kann, bis das Hülsenende am Anschlag 43 ansteht.

Durch entsprechende Montage des Haltestückes 40 am Bohrerschaft 23 kann also vor dem Bohren die Bohrtiefe relativ zur Schneidekante eingestellt, das heisst vorselektioniert werden. Zur Anzeige der eingestellten Bohrtiefe ist mit Vorteil am äussersten Ende des Bohrerschaftes 23 eine Markierung 44 angeordnet.

Selbstverständlich kann das Haltestück 40 auch fest (nicht verschiebbar) auf dem Bohrerschaft 23 montiert sein, wodurch die Vorrichtung für eine bestimmte Bohrtiefe (ab Schneidekante) reserviert ist.

Die Vorrichtung gemäss Figur 4 weist im Gegensatz zur Vorrichtung gemäss Figur 1 am Bohrerschaft und an der Innenoberfläche der Hülse kein Gewinde zum drehenden Vorschieben des Bohrers auf. Es zeigt sich, dass auf das den Vorschub des Bohrers dosierend wirkende Gewinde verzichtet werden kann. Seine Führungsfunktion wird in diesem Falle vorteilhafterweise durch einen in der Hülse angeordneten Führungsring 28 übernommen.

Figur 5 zeigt ein Hülsenende mit Schneidekante 11, auf dem ein Anschlagstück 50 beispielsweise mit einer Fixierschraube 41 montiert ist. Wenn dieses Hülsenende in eine Gewebeoberfläche gepresst wird, bleibt die der Schneidekante 11 zugewandte Stirnfläche 51 des Anschlagstücks 50 auf der Gewebeoberfläche stehen und limitiert dadurch die Tiefe, in die die Schneidekante in das Gewebe eingepresst werden kann. Durch entsprechende Montage des Anschlagstückes ist die Einpresstiefe vorselektionierbar und an einer entsprechenden Markierung 30 ablesbar.

Für das Anschlagstück 50 gilt wie für das Haltestück 40, dass es auch fest montiert sein kann, wodurch die Vorrichtung für eine bestimmte Einpresstiefe reserviert ist.

Damit das Anschlagstück die Sicht des Chirurgen beim Einpressen der Schneidekante in das Gewebe nicht unvorteilhaft verdeckt, ist es vorteilhafterweise nicht als geschlossener Ring um die Hülse ausgestaltet, sondern beispielsweise als offener Ring. Ein derartiges Anschlagstück 50' ist in der Figur 6 als Draufsicht dargestellt.

Ein fest montiertes Anschlagstück kann auch durch eine entsprechende Ausgestaltung der Hülsenwand ersetzt werden, wie dies in der Figur 7 dargestellt ist. Die Anschlagfläche 51 ist in diesem Falle eine stufenförmige Vergrösserung des Aussenduchmessers der Hülse.

## Patentansprüche

1. Vorrichtung zur Herstellung von endochondralen oder osteochondralen Bohrungen, mit einer im wesentlichen hohlzylinderförmige Hülse (10) die mindestens im Bereich ihres einen Endes einen konstanten Innendurchmesser hat und die an diesem Ende in eine Schneidekante (11) ausläuft, und mit einem im Bereich des genannten Endes der Hülse (10) angeordneten Bohrer, der in der Hülse (10) drehbar angeordnet ist, **dadurch gekennzeichnet, dass** der Bohrer ein Flachbohrer (20) ist, der in der Hülse (10) unter Drehung axial derart verschiebbar ist, dass sein flaches, distales Ende (21) im Bereich der Schneidekante (11) in Positionen innerhalb der Hülse (10) und ausserhalb der Hülse (10) bringbar ist.

2. Vorrichtung nach Anspruch 1, **dadurch gekennzeichnet, dass** an dem Flachbohrer (20) ein den Flachbohrer axial verlängernder Bohrerschaft (23) angeordnet ist, der auf der der Schneidekante (11) entgegengesetzten Seite aus der Hülse (10) ragt.

3. Vorrichtung nach einem der Ansprüche 1 oder 2, **dadurch gekennzeichnet, dass** der Bohrerschaft (23) in der Hülse (10) durch ein Gewinde (24) oder durch einen Führungsring (28) geführt ist.

4. Vorrichtung nach Anspruch 2 oder 3, **dadurch gekennzeichnet, dass** auf dem aus der Hülse (10) ragenden Teil des Bohrerschaftes (23) ein Haltestück (40) montiert ist.

5. Vorrichtung nach Anspruch 4, **dadurch gekennzeichnet, dass** der Vorschub des Flachbohrers (20) in der Hülse (10) durch das Haltestück (40) begrenzt ist.

6. Vorrichtung nach Anspruch 4 oder 5, **dadurch gekennzeichnet, dass** das Haltestück (40) verschiebbar auf dem Bohrerschaft (23) montiert ist.

7. Vorrichtung nach einem der Ansprüche 1 bis 6, **dadurch gekennzeichnet, dass** die Hülse (10) zur Begrenzung einer Einpresstiefe eine von der Schneidekante (11) beabstandete Anschlagfläche (51) aufweist.

8. Vorrichtung nach Anspruch 7, **dadurch gekennzeichnet, dass** die Anschlagfläche (51) an einem auf der Hülse (10) montierbaren Anschlagstück (50) angeordnet ist.

9. Vorrichtung nach Anspruch 8, **dadurch gekennzeichnet, dass** das Anschlagstück (50) verschiebbar auf der Hülse (10) montierbar ist.

10. Vorrichtung nach einem der Ansprüche 1 bis 9, **dadurch gekennzeichnet, dass** im Bereich der Schneidekante (11) an der Aussenoberfläche der Hülse (10) und am aus dem anderen Ende der aus der Hülse (10) ragenden Bohrerschaft (23) Markierungen (30,31,44) zur Bestimmung der Einpresstiefe der Schneidekante (11) in ein Gewebe und zur Bestimmung der Bohrerposition relativ zur Schneidekante (11) vorgesehen sind.

## Claims

1. Apparatus for the production of endochondral or osteochondral bores comprising a substantially hollow cylindrical sleeve (10) which has a constant inner diameter at least in the region of its one and which runs out into a cutting edge (11) at this end, and comprising a borer which is arranged in the region of the named end of the sleeve (10) and is rotatably arranged in the sleeve (10), **characterised in that** the borer is a flat borer (20) which can be axially displaced in the sleeve (10) while being rotated in such a manner that its flat, distal end (21) can be brought in the region of the cutting edge (11) into positions inside the sleeve (10) and outside the sleeve (10).

2. Apparatus in accordance with claim 1, **characterised in that** a borer shaft (23) which axially extends the flat borer is arranged at the flat borer (20) and projects out of the sleeve (10) at the side opposite to the cutting edge (11).

3. Apparatus in accordance with claim 1 or claim 2 **characterised in that** the borer shaft (23) is guided in the sleeve (10) by a thread (24) or by a guide ring (28).

4. Apparatus in accordance with claim 2 or claim 3 **characterised in that** a holder piece (40) is mounted on the part of the borer shaft (23) projecting out of the sleeve (10).

5. Apparatus in accordance with claim 4 **characterised in that** the forward thrust of the flat borer (20) in the sleeve (10) is limited by the holder piece (40).

6. Apparatus in accordance with claim 4 or claim 5 **characterised in that** the holder piece (40) is displaceably mounted on the borer shaft (23).

7. Apparatus in accordance with one of the claims 1 to 6 **characterised in that** the sleeve (10) has an abutment surface (51) which is spaced with respect to the cutting edge (11) for the limiting of a pressing-in depth.

8. Apparatus in accordance with claim 7 **characterised in that** the abutment surface (51) is arranged at an abutment piece (50) which can be mounted on the sleeve (10).

9. Apparatus in accordance with claim 8 **characterised in that** the abutment piece (50) is displaceably mounted on the sleeve (10).

10. Apparatus in accordance with one of the claims 1 to 9 **characterised in that** markings (30, 31, 44) are provided in the region of the cutting edge (11) at the outer surface of the sleeve (10) and at the borer shaft (23) projecting out of the outer end of the sleeve (10) for the determination of the pressing-in depth of the cutting edge (11) into a tissue and for the determination of the borer position relative to the cutting edge (11).

## Revendications

1. Dispositif pour réaliser des trous endochondraux et ostéochondraux, avec un manchon (10) sensiblement en forme de cylindre creux qui présente au moins au voisinage d'une extrémité un diamètre intérieur constant et qui se termine à cette extrémité par une arête coupante (11), et avec un foret disposé au voisinage de l'extrémité précitée du manchon (10), qui est disposé d'une manière tournante dans le manchon (10), **caractérisé en ce que** le foret est un foret plat (20) qui est déplaçable axialement dans le manchon (10), en tournant, de façon que son extrémité distale plate (21) puisse être amenée au voisinage de l'arête de coupe (11) dans des positions à l'intérieur du manchon (10) et à l'extérieur du manchon (10).

2. Dispositif selon la revendication 1, **caractérisé en ce qu'**il est disposé au foret plat (20) une queue de foret (23) prolongeant axialement le foret plat qui fait saillie au côté opposé à l'arête de coupe (11) du manchon (10).

3. Dispositif selon l'une des revendications 1 ou 2, **caractérisé en ce que** la queue de foret (23) est guidée dans le manchon (10) par un filetage (24) ou par une bague de guidage (28).

4. Dispositif selon la revendication 2 ou 3, **caractérisé en ce qu'**il est monté sur la partie de la queue de foret (23) dépassant du manchon (10) une pièce de retenue (40).

5. Dispositif selon la revendication 4, **caractérisé en ce que** l'avance du foret plat (20) dans le manchon (10) est limitée par la pièce de retenue (40).

6. Dispositif selon la revendication 4 ou 5, **caractérisé en ce que** la pièce de retenue (40) est montée d'une manière déplaçable sur la queue de foret (23).

7. Dispositif selon l'une des revendications 1 à 6, **caractérisé en ce que** le manchon (10), pour limiter une profondeur d'enfoncement, présente une face de butée (51) espacée de l'arête de coupe (11).

8. Dispositif selon la revendication 7, **caractérisé en ce que** la face de butée (51) est montée sur une pièce de butée (50) pouvant être montée sur le manchon (10).

9. Dispositif selon la revendication 8, **caractérisé en ce que** la pièce de butée (50) peut être montée d'une manière déplaçable sur le manchon (10).

10. Dispositif selon l'une des revendications 1 à 9, **caractérisé en ce que** sont prévus au voisinage de l'arête de coupe (11) à la face supérieure extérieure du manchon (10) et à la queue de foret (23) dépassant de l'autre extrémité du manchon (10) des marquages (30, 31, 44) pour définir la profondeur d'enfoncement de l'arête de coupe (11) dans un tissu et pour déterminer la position du foret relativement à l'arête de coupe (11).
